# FASCICULE DE BREVET EUROPEEN

(11) **EP 0 436 712 B1**
(45) Date de publication et mention de la délivrance du brevet: **08.11.1995**
(21) Numéro de dépôt: 90912569.2
(22) Date de dépôt: 31.07.1990
(51) Int. Cl.: A61L 2/04, A61L 25/00

(54) **PROCEDE DE PASTEURISATION ET COLLE PASTEURISEE POUR REUNIR DES TISSUS HUMAIN OU ANIMAL**
PASTEURISIERENSVERFAHREN UND PASTEURISIERTER KLEBER ZUM BINDEN VON MENSCHLICHEN ODER TIERISCHEN GEWEBEN
PASTEURISATION METHOD UND PASTEURISED ADHESIVE FOR SECURING HUMAN OR ANIMAL TISSUES

(30) Priorité: 01.08.1989 FR 8910355
(43) Date de publication de la demande: 17.07.1991
(73) Titulaire: FONDATION NATIONALE DE TRANSFUSION SANGUINE, 75739 Paris Cédex 15 (FR)
(72) Inventeur: CHABBAT, Jacques, F-75017 Paris (FR); STEINBUCH, Marion, F-78180 Voisins-le-Bretonneux (FR)
(74) Mandataire: Warcoin, Jacques
(86) Numéro de dépôt international: FR9000577
(87) Numéro de publication internationale: WO9101762

(56) Documents cités:
- EP-A- 0 011 739
- EP-A- 0 035 204
- EP-A- 0 037 078
- EP-A- 0 103 196
- EP-A- 0 183 674

## Description

La présente invention se rapporte à une colle pasteurisée pour réunir des tissus humain ou animal.

Il est depuis longtemps connu d'utiliser des substances favorisant la coagulation du sang pour stopper des hémorragies ou cicatriser des blessures.

Dans les premières propositions qui ont été faites dans ce domaine, on a tout d'abord utilisé des tampons ou des plaquettes de fibrine puis, par la suite on a réalisé des collages de tissus à l'aide de plasma sanguin.

Ce type de colle est de façon générale un concentré de facteurs de l'hémostase coagulable par la thrombine riche en fibrinogène.

Elle est constituée de deux composants essentiels qui sont mélangés extemporanément lors de l'utilisation. Le premier composant est constitué d'un mélange de protéines extraites du plasma humain : fibrinogène, fibronectine, et facteurs XIII (ou facteurs stabilisateurs de la fibrine). Le deuxième composant est constitué de thrombine calcique qui peut être d'origine animale, bovine par exemple.

Afin d'éviter tout risque de contamination virale lors de l'application de cette colle, il est nécessaire d'effectuer, au cours de sa préparation, une étape d'inactivation virale du premier composant à base de protéines d'origine plasmatique. La pasteurisation qui est une méthode appropriée pour ce type d'opération pose en fait des problèmes en raison de l'instabilité thermique de certaines protéines présentes dans ce premier composant.

EP 103196 décrit un procédé de pasteurisation de solutions de fibrinogène en présence de concentrations élevées de calcium, qui entraîne la formation d'un précipité insoluble de fibrine.

EP 37078 décrit la pasteurisation de solutions aqueuses de facteur XIII ne contenant pas de fibrinogène en présence d'au moins 10% d'un sel d'un acide carboxylique en C₃-C₁₀.

EP 35204 décrit un procédé de pasteurisation d'un mélange de protéines plasmatiques dans lequel la teneur en fibrinogène est inférieure à 60% des protéines totales, en présence d'un polyol.

EP 11739 concerne un procédé de purification de facteur XIII à partir de placenta, suivie d'une pasteurisation du produit obtenu.

Le but de la présente invention est de proposer une nouvelle colle pasteurisée essentiellement caractérisée par le procédé d'obtention du premier composant extrait du plasma humain, ce procédé incluant une étape d'inactivation virale.

Plus particulièrement, la présente invention concerne un procédé de pasteurisation d'un mélange de protéines contenant principalement du fibrinogène par chauffage dudit mélange dans des conditions assurant la pasteurisation, caractérisé en ce que :
a) un cryoprécipité de plasma humain est mis en solution puis précipité en présence d'héparine,
b) le précipité obtenu est remis en solution et constitue le mélange à pasteuriser,
c) ledit mélange est pasteurisé en présence d'un monosaccharide et d'un sucre alcoolique, à 60° C pendant 10 heures, ou dans des conditions de pasteurisation équivalentes,
et en ce que ledit mélange est exempt d'ion calcium, avec la condition que la teneur en fibrinogène dans le mélange est supérieure à 60%.

De préférence et à la différence de nombreux procédés de la technique antérieure, notamment le procédé décrit dans EP-B-0 103 196, le mélange de protéines pasteurisé ne contient pas d'ion calcium et est, de préférence, exempt d'inhibiteur de la thrombine.

Dans l'application préférée de ce procédé, le mélange de protéines contient également du facteur XIII plasmatique et peut être obtenu à partir d'un cryoprécipité de plasma humain mis en solution et précipité en présence d'héparine, le précipité obtenu remis en solution constitue le mélange à pasteuriser.

Les inventeurs ont en effet mis en évidence que la présence d'un monosaccharide et d'un sucre alcoolique, lors de la pasteurisation de la solution protéique, permet de stabiliser lesdites protéines et par conséquent de préserver leurs activités biologiques.

Les concentrations en monosaccharide et sucre alcoolique utilisées pour stabiliser les protéines dépendent, bien entendu, de la nature, la concentration des protéines dans la solution protéique et de la nature même du monosaccharide et du sucre alcoolique utilisés et sont en général de 1000 et 1400 g pour le monosaccharide et entre 200 et 400 g pour le sucre alcoolique rajoutés par litre de solution protéique.

Selon un mode de réalisation préféré de l'invention, on utilise de préférence le glucose comme monosaccharide et le sorbitol comme sucre alcoolique à des concentrations de 1 200 g pour le glucose et de 300 g pour le sorbitol rajoutés par litre de solution protéique.

Selon l'invention, on pasteurise la solution protéique à une température de 60°C pendant 10 heures ou dans des conditions de pasteurisation équivalentes, c'est-à-dire des températures et des durées de traitement suffisantes pour permettre de neutraliser toute trace de virus présent dans le produit, en particulier le virus de l'hépatite. Cette pasteurisation sera effectuée dans des conditions proches des conditions physiologiques afin de ne pas perturber les protéines. Le pH utilisé sera ainsi de préférence égal à 8.

L'invention concerne également une colle pour réunir les tissus humain ou animal de type comportant deux composants :
a) un mélange de protéines extraites du plasma humain contenant principalement du fibrinogène et du facteur XIII et,
b) de la thrombine calcique,
mélangés extemporanément au moment du collage, caractérisée en ce que le mélange de protéines contenant principalement du fibrinogène et du facteur XIII est pasteurisé par le procédé décrit précédemment.

La description détaillée d'un mode de réalisation de l'invention, donnée ci-dessous à titre non limitatif permettra de mettre en évidence d'autres caractéristiques de l'invention.

Le cryoprécipité est obtenu par des techniques connues à partir de plasma humain décongelé à une température de 0 à 4°C, puis centrifugé. Le cryoprécipité est ensuite remis en solution dans un tampon Tris à pH 7. La concentration en protéines est alors de 15 à 35 g/l.

Le mélange de protéines est précipité de la solution obtenue par l'héparine. On utilisera de préférence de 10 à 40 U/ml d'héparine et une température de 10 à 35°C.

Le précipité obtenu est alors récupéré par centrifugation et le surnageant élimine.

Le précipité obtenu est ensuite solubilisé dans un tampon à pH 8 contenant des agents stabilisants en particulier des amino acides tels que glycine et arginine ainsi que des inhibiteurs de la fibrinolyse tels que l'acide ε aminocaproïque et l'aprotinine (inhibiteur de la plasmine). La concentration protéique de la solution est dans ce cas de 40 à 70 g/l. A ce stade, il est possible d'envisager une étape de filtration clarifiante pour éliminer les insolubles.

Ce n'est donc qu' au moment de l'étape de pasteurisation que les stabilisants de pasteurisation à proprement dits, c'est-à-dire le monosaccharide et le sucre alcoolique, sont introduits. La solution protéique contenant ces stabilisants est alors chauffée à une température de 60°C pendant 10 heures.

Afin d'éliminer les stabilisants après le traitement d'inactivation virale et de concentrer les protéines, on pourra utiliser des méthodes usuelles de type précipitation, dialyse ou filtration par exemple.

Ainsi, la solution pasteurisée pourra être diluée dans un tampon à un pH situé entre 6 et 7 contenant du NaCl 0,015 M - citrate trisodique sur 5mM acide ε aminocaproïque 25 mM et arginine 17 mM. La solution diluée dans un rapport 1/5 à 1/10 (V/V) est refroidie à une température comprise entre -1° et -5°. Les protéines sont précipitées par addition d'alcool à 10 % final en maintenant une température inférieure à -1°C et supérieure à -5°C, le précipité récupéré après centrifugation et le surnageant éliminé.

Le culot de centrifugation est ensuite remis en solution avec un tampon à pH 7,5 contenant du NaCl 0,06 M - citrate trisodique 1 mM - glycine 60 mM - acide ε aminocaproïque 20 mM et arginine 20 mM. La solution obtenue est dialysée contre le même tampon puis concentrée pour atteindre une concentration protéique de 30 à 40 g/l.

A ce stade, il peut être rajouté des agents favorisant la solubilité du produit après lyophilisation tels que du polysorbate et/ou caprylate de sodium. La solution est alors filtrée stérilement à l'aide de filtres ayant une porosité de 0,2 µm répartie en flacons puis lyophilisée.

Le lyophilisat peut être repris avec une solution d'eau PPI pouvant contenir un inhibiteur de la plasmine (aprotinine par exemple). La solution obtenue doit contenir au minimum une concentration de fibrinogène de 70 g/l.

Le produit reconstitué contient du fibrinogène, de la fibronectine dans les proportions suivantes : respectivement 65 à 85 % et 7 à 14 % rapportées aux protéines totales. Il contient également du FXIII ainsi que de l'héparine (50 à 200 U/ml).

L'exemple donné ci-dessous à titre non limitatif permettra de mettre en évidence d'autres caractéristiques de la présente invention.

### EXEMPLE 1 :

A partir de 280 l de plasma, la cryoprécipitation a permis de récupérer 2 kg de précipité (cryoprécipité). Les protéines entrant dans la composition de la colle sont obtenues après remise en solution de ce précipité (1 kg/4 litres ) dans un tampon tris 20 mM pH 7, suivi d'une précipitation à la température de 25°C après addition d'héparine à 32 U/ml. La suspension obtenue est centrifugée, le culot (environ 1 kg) est récupéré puis remis en solution dans du citrate trisodique 70 mM -glycine 0,1 M -NaCl 0,03 M - acide ε aminocaproïque 50 mM - arginine 50 mM et aprotinine 100 U/ml à pH 8 (1 kg/1 litre). On procède alors à un chauffage liquide 10 heures à 60°C après avoir ajouté des stabilisants de protéines, à savoir 2400 g de glucose et 600 g de sorbitol à 2 litres de solution pour un volume final d'environ 4 litres (compte tenu de la dilution due à l'addition des sucres). Après pasteurisation la solution est diluée 10 fois dans du citrate 5 mM-NaCl 0,051 M - acide ε aminocaproïque 25 mM - arginine 17 mM pH 7. La solution obtenue est alors précipitée à l'alcool à 10 % final à une température comprise entre -2 et -3°C. Les protéines précipitées sont récupérées dans le culot de centrifugation. Celui-ci est ensuite remis en solution dans du citrate trisodique 1 mM - NaCl 60 mM - acide ε aminocaproïque 20 mM - glycine 60 mM pH 7,5.

Les protéines sont ensuite concentrées jùsqu'à un taux de 27 - 30 g/l pour un volume final de 1,5 l. Le produit est ensuite réparti en flacons après filtration stérilisante puis lyophilise. La concentration en protéines coagulables peut être modulée en fonction du volume de reconstitution du lyophilisat. Le produit obtenu présente, pour un taux de protéines de 110 g/l, 77 g/l de fibrinogène coagulable, 12 g/l de fibronectine et un taux d'aprotinine de 2 000 KIU/ml (solvant utilisé pour la reconstitution du lyophilisat).

## Revendications

1. Procédé de pasteurisation d'un mélange de protéines contenant principalement du fibrinogène par chauffage dudit mélange dans des conditions assurant la pasteurisation, caractérisé en ce,
a) un cryoprécipité de plasma humain est mis en solution puis précipité en présence d'héparine,
b) le précipité obtenu est remis en solution et constitue le mélange à pasteuriser,
c) ledit mélange est pasteurisé en présence d'un monosaccharide et d'un sucre alcoolique, à 60° C pendant 10 heures, ou dans des conditions de pasteurisation équivalentes,
et en ce que ledit mélange est exempt d'ion calcium, avec la condition que la teneur en fibrinogène dans le mélange est supérieure à 60%.

2. Procédé selon la revendication 1, caractérisé en ce que le mélange est exempt d'inhibiteur de la thrombine.

3. Procédé selon la revendication 1, caractérisé en ce que le mélange contient du facteur XIII plasmatique.

4. Procédé selon l'une des revendications 1 à 3, caractérisé en ce que la pasteurisation est conduite en présence de glucose et de sorbitol.

5. Procédé selon l'une des revendications 1 à 4, caractérisé en ce que la quantité de monosaccharide utilisée est comprise entre 1000 et 1400 g à ajouter par litre de solution et la quantité de sucre alcoolique entre 200 et 400 g à ajouter par litre de solution.

6. Procédé selon la revendication 5, caractérisé en ce que les quantités de glucose et de sorbitol à ajouter par litre de solution sont respectivement de 1200 g/l et de 300 g/l.

7. Procédé selon l'une des revendications 1 à 6, caractérisé en ce que la précipitation en présence d'héparine est effectuée à une température de 10 à 35° C.

8. Procédé selon l'une des revendications 1 à 7, caractérisé en ce que l'étape de pasteurisation est conduite à des concentrations de 40 à 70 g/l de protéines.

9. Procédé selon l'une des revendications 1 à 8, caractérisé en ce que le précipité avant pasteurisation contient d'autres agents stabilisants et des inhibiteurs de la fibrinolyse.

10. Colle pour réunir des tissus humains ou animal du type comportant deux composants :
a) un mélange de protéines extraites du plasma humain contenant principalement du fibrinogène et du facteur XIII et,
b) de la thrombine calcique,
mélangés extemporanément au moment du collage, caractérisée en ce que le mélange de protéines contenant principalement du fibrinogène et du facteur XIII est pasteurisé par le procédé selon l'une des revendications 1 à 9.

## Claims

1. Process for the pasteurization of a mixture of proteins principally containing fibrinogen, by heating the said mixture under conditions effecting pasteurization, characterized in that
a) a cryoprecipitate of human plasma is dissolved and then precipitated in the presence of heparin,
b) the precipitate obtained is redissolved and constitutes the mixture to be pasteurized,
c) the said mixture is pasteurized in the presence of a monosaccharide and a sugar alcohol at 60°C for 10 hours, or under equivalent pasteurization conditions,
and in that the said mixture is free from calcium ion, on condition that the fibrinogen content in the mixture is greater than 60%.

2. Process according to Claim 1, characterized in that the mixture is free from thrombin inhibitor.

3. Process according to Claim 1, characterized in that the mixture contains plasma factor XIII.

4. Process according to one of Claims 1 to 3, characterized in that the pasteurization is performed in the presence of glucose and sorbitol.

5. Process according to one of Claims 1 to 4, characterized in that the quantity of monosaccharide used is between 1,000 and 1,400 g to be added per litre of solution, and the quantity of sugar alcohol between 200 and 400 g to be added per litre of solution.

6. Process according to Claim 5, characterized in that the quantities of glucose and sorbitol to be added per litre of solution are 1,200 g/l and 300 g/l, respectively.

7. Process according to one of Claims 1 to 6, characterized in that the precipitation in the presence of heparin is performed at a temperature of 10 to 35°C.

8. Process according to one of Claims 1 to 7, characterized in that the pasteurization step is performed at protein concentrations of 40 to 70 g/l.

9. Process according to one of Claims 1 to 8, characterized in that the precipitate before pasteurization contains other stabilizing agents and fibrinolysis inhibitors.

10. Glue for joining human or animal tissues, of the type containing two components:
a) a mixture of proteins extracted from human plasma, principally containing fibrinogen and factor XIII, and
b) thrombin/calcium,
mixed as required at the time of sticking, characterized in that the mixture of proteins principally containing fibrinogen and factor XIII is pasteurized by the process according to one of Claims 1 to 9.

## Patentansprüche

1. Verfahren zur Pasteurisierung einer Protein-Mischung, die hauptsächlich Fibrinogen enthält, durch Erwärmen der genannten Mischung unter Bedingungen, die eine Pasteurisierung gewährleisten, dadurch gekennzeichnet, daß man
a) ein Kryopräzipitat von Humanplasma in Lösung bringt und dann in Gegenwart von Heparin ausfällt,
b) den erhaltenen Niederschlag wieder in Lösung bringt, wobei diese die zu pasteurisierende Mischung darstellt, und
c) die genannte Mischung in Gegenwart eines Monosaccharids und eines alkoholischen Zuckers bei 60°C 10 h lang oder unter äquivalenten Pasteurisierungsbedingungen pasteurisiert,
wobei die genannte Mischung frei von Calciumionen ist mit der Bedingung, daß der Gehalt an Fibrinogen in der Mischung über 60 % liegt.

2. Verfahren nach Anspruch 1, dadurch gekennzeichnet, daß die Mischung frei von einem Thrombin-Inhibitor ist.

3. Verfahren nach Anspruch 1, dadurch gekennzeichnet, daß die Mischung den Plasma-Faktor XIII enthält.

4. Verfahren nach einem der Ansprüche 1 bis 3, dadurch gekennzeichnet, daß die Pasteurisierung in Gegenwart von Glucose und Sorbit durchgeführt wird.

5. Verfahren nach einem der Ansprüche 1 bis 4, dadurch gekennzeichnet, daß die verwendete Monosaccharid-Menge, die pro Liter Lösung zugegeben werden soll, zwischen 1000 und 1400 g liegt, und daß die Alkoholzucker-Menge, die pro Liter Lösung zugegeben werden soll, zwischen 200 und 400 g liegt.

6. Verfahren nach Anspruch 5, dadurch gekennzeichnet, daß die Glucose- und Sorbit-Mengen, die pro Liter Lösung zugegeben werden sollen, jeweils 1200 g/l bzw. 300 g/l betragen.

7. Verfahren nach einem der Ansprüche 1 bis 6, dadurch gekennzeichnet, daß die Ausfällung in Gegenwart von Heparin bei einer Temperatur von 10 bis 35°C durchgeführt wird.

8. Verfahren nach einem der Ansprüche 1 bis 7, dadurch gekennzeichnet, daß die Pasteurisierungsstufe bei Protein-Konzentrationen von 40 bis 70 g pro Liter durchgeführt wird.

9. Verfahren nach einem der Ansprüche 1 bis 8, dadurch gekennzeichnet, daß der Niederschlag vor der Pasteurisierung andere Stabilisierungsmittel und Fibrinolyse-Inhibitoren enthält.

10. Klebstoff (Leim) vom Zwei-Komponenten-Typ zum Verkleben (Verbinden) von Human- oder Tiergeweben, der umfaßt:
a) eine Mischung von Proteinen, die aus Humanplasma extrahiert worden sind, das hauptsächlich Fibrinogen und den Faktor XIII enthält, und
b) Calciumthrombin,
die beim Verkleben in situ miteinander gemischt werden, dadurch gekennzeichnet, daß die Protein-Mischung, die hauptsächlich Fibrinogen und den Faktor XIII enthält, nach dem Verfahren nach einem der Ansprüche 1 bis 9 pasteurisiert worden ist.
